# EUROPÄISCHE PATENTANMELDUNG

(11) **EP 4 427 767 A1**
(43) Veröffentlichungstag der Anmeldung: **11.09.2024**
(21) Anmeldenummer: 23161352.2
(22) Anmeldetag: 10.03.2023
(51) Int. Cl.: A61M 1/34, A61M 1/36, A61M 1/00

(54) **VORRICHTUNG UND VERFAHREN ZUR EXTRAKORPORALEN BLUTWÄSCHE UNTER ABTRENNUNG VON ANGIOTENSIN-SPEZIFISCHEN ANTIKÖRPERN**

(71) Anmelder: Klingenberg, Georg, 13505 Berlin (DE)
(72) Erfinder: Klingenberg, Georg, 13505 Berlin (DE)
(74) Vertreter: Gulde & Partner

(57) **Zusammenfassung**

Die vorliegende Erfindung beschreibt eine Vorrichtung zur extrakorporalen Blutwäsche mit dem Ziel, bestimmte Antikörper aus dem menschlichen Blut zu entfernen. Die zu entfernenden Antikörper richten sich gegen Angiotensine. Die in diesem Dokument beschriebene Vorrichtung hat das Ziel, diese speziellen Antikörper aus dem Blut zu entfernen und auf diese Weise die oben beschriebenen Krankheiten und ihre Folgen zu lindern. Beschrieben werden auch Verfahren zur Durchführung der extrakorporalen Blutwäsche unter Anwendung der beschriebenen Vorrichtung.

## Beschreibung

Die vorliegende Erfindung beschreibt eine Vorrichtung zur extrakorporalen Blutwäsche (Apherese) mit dem Ziel, bestimmte Antikörper aus dem menschlichen Blut zu entfernen. Die zu entfernenden Antikörper richten sich gegen Angiotensine.

Es gibt Hinweise, wonach manche Menschen nach bestimmten Vireninfektionen (zum Beispiel SARS-CoV2) Antikörper bilden, die gegen verschiedene Angiotensine gerichtet sind. Derartige Antikörper können langanhaltende Störungen verursachen und sind möglicherweise eine Ursache für sogenannte "Long-Covid-Erkrankungen" und ähnliche krankhafte Erscheinungsformen nach viralen Infektionen, die in einer Teilmenge der Infizierten zu beobachten sind. Die gebildeten Antikörper sind möglicherwese nicht nur spezifisch gegen Angiotensine gerichtet, sondern richten sich auch gegen bestimmte virale Proteine, z.B. das Spike-Protein. Die in diesem Dokument beschriebene Vorrichtung hat das Ziel, diese speziellen Antikörper aus dem Blut zu entfernen und auf diese Weise die oben beschriebenen Krankheiten und ihre Folgen zu lindern. Beschrieben werden auch Verfahren zur Durchführung der extrakorporalen Blutwäsche unter Anwendung der beschriebenen Vorrichtung.

Die vorliegende Erfindung basiert auf einer Technologie, die darauf abzielt, Antikörper, welche vom Körper in Folge einer Infektion z.B. mit Corona-Viren (im Speziellen SARS-CoV1 & SARS-CoV2), aber auch anderen Viren gebildet werden und dann eine Spezifität gegen das Angiotensin II (Angiotensinogen, Angiotensin I [Angiotensin 1-9 & Angiotensin 1-7 sind ähnliche Strukturen und würden u.a. auch durch die Antikörper gebunden werden]) bilden, aus Körperflüssigkeiten zu entfernen. Diese Virus-induzierte Spezifität der Antikörper zum körpereigenen Angiotensin II (aAB_{angII}) u.a. hat womöglich zur Folge, dass der durch die Bindung des freien u.a. (s.o.) Angiotensin II eine Störung des Renin-Angiotensin-Aldosteron-Systems (RAAS) induziert wird, die akut lebensbedrohlich sein kann und/oder auch. langanhaltend sein kann. Diese Störung könnte eine Ursache von "Long-Covid" sein. Die vorliegende Erfindung zielt darauf ab, die gebildeten Anti-Angiotensin-Antikörper aus dem Körper zu entfernen, z.B. durch eine extrakorporale Behandlung der von Körperflüssigkeiten, insbesondere Blut und Liquor. Die Körperflüssigkeit wird dabei mit einem festphasenfixierten Angiotensin bzw, einem der unten genannten Derivate in Kontakt gebracht. Dabei binden sich die Antikörper an das Angiotensin II, Angiotensin I oder Angiotensinogen oder einem Derivat bzw. einer Struktur, welche die Sequenz von ANG I/II enthält, wobei das Angiotensin bzw. Derivat hiervon mittels eines Linkers, ggf, unter Verwendung eines Spacers, an eine immobile Festphase gekoppelt ist. Das Linker/Spacer-System kann starr oder flexibel sein. Wichtig ist, dass es die Bindung des Antikörpers an das Angiotensin ermöglicht und nicht sterisch beeinträchtigend wirkt. Die immobile Festphase ist eine hydrophile oder hydratisierte Membran, wie sie auch aus zahlreichen anderen Verfahren bekannt ist.

Nach Blutentnahme (analog zur klassischen Blutwäsche) können aus dem Blutplasma die Angiotensin I/II/-ogen Autoantikörper entfernt werden und das Serum, Plasma bzw. Blut dem Patienten wieder verabreicht werden. Durch das differentielle und spezifische Entfernen der Angiotensin I/II/-ogen Autoantikörper kann eine schonende (keine indifferentielle Blutwäsche) und quantitativ regelbare (Variation der Blutfilterleistung anhand von diversen physiologischen Faktoren) Entfernung durchgeführt werden, wodurch sich dann wieder nach einem gewissen Zeitraum ein Normalzustand der RAAS einstellen kann. [Die Membran kann beispielsweise einfach in handelsübliche Plasmaspende Einheiten implementiert werden.] Das Angiotensin I/II/-ogen wird dabei üblicherweise C-Terminal, N-Terminal oder in Mischform (C-&N-Terminal) an die Membran (dessen Oberflächen optimiert ist), den Linker/Spacer oder den Anchor gebunden (ggf. flexibel oder starr). Alternativ möglich ist eine Bindung des Angiotensins über die funktionellen Gruppen des Angiotensins, insbesondere über die Seitengruppen der Aminosäuren. Dabei sind kovalente Bindungen bevorzugt, möglich sind aber auch ionische, dative, Chelat-, Metallbindungen oder jede andere Art der Immobilisierung.

Bei Bedarf kann der Abbau durch Amino- & Carboxy Exoproteasen durch C-terminale und N-Terminale cappings verhindert werden, um eine langanhaltende hohe Effizienz des Membranmaterials zu erhalten (Haltbarkeit Lagerung & Haltbarkeit bei Prozessbelastung).

Die Erfindung betrifft daher eine Vorrichtung zur extrakorporalen Behandlung von Körperflüssigkeiten durch Abtrennung von Angiotensin-spezifischen Antikörpern aus dem Blutplasma und oder Liquor, enthaltend mindestens eine Kartusche mit festphasengebundenem Angiotensinen sowie ein Verfahren zur extrakorporalen Blutwäsche (Apherese) unter Verwendung dieser Vorrichtung.

Unter Angiotensin-spezifischen Antikörpern (auch: Anti-Angiotensin Antikörper) werden im Rahmen dieser Beschreibung Antikörper verstanden, die sich spezifisch an Angiotensine binden, insbesondere an Angiotensin I (Angiotensin 1 bis 10), Angiotensin 1 bis 9, Angiotensin II (Angiotensin 1 bis 8), Angiotensin 1 bis 7, Angiotensin III (Angiotensin 2 bis 8) und Angiotensin IV (Angiotensin 3 bis 8) oder an Angiotensinogen. Unter Angiotensin-spezifischen Antikörpern werden auch solche Antikörper verstanden, nicht nur an Angiotensin bzw. Angiotensinogen binden, sondern die eine Co-spezifität aufweisen und sich daher auch gegen andere Strukturen richten, insbesondere gegen virale Proteine, z.B. das sogenannte Spike Protein von SARS-CoV2.

Die Vorrichtung enthält zunächst eine oder mehrere Angiotensine, die an eine Festphase gebunden sind. Angiotensine sind in der Literatur ausreichend beschrieben. Es handelt sich hierbei um Peptidhormone, die Wirkung auf Blutgefäße haben und auf diese Weise den Blutdruck beeinflussen. Zusätzlich können sie Einfluss auf die Nebennieren und hierüber insbesondere auf den Aldosteronhaushalt haben. Als Angiotensine sind insbesondere bekannt Angiotensin I (Angiotensin 1 bis 10), Angiotensin 1 bis 9, Angiotensin II (Angiotensin 1 bis 8), Angiotensin 1 bis 7, Angiotensin III (Angiotensin 2 bis 8) und Angiotensin IV (Angiotensin 3 bis 8). Die erfindungsgemäße Vorrichtung kann einzelne Angiotensine oder eine Mischung verschiedener Angiotensine enthalten. Die Sequenzen der genannten Angiotensine sind in der Fachliteratur beschrieben und bedürfen daher an dieser Stelle keiner Wiederholung. Erfindungsgemäß sind auch Angiotensin-Derivate, mit Teilen der o.g. Angiotensin-Sequenzen sowie Derivate, bei denen die Sequenzabfolge invertiert und/oder bei denen Aminosäuren ausgetauscht wurden sowie glykolisierte Derivate. Weiterhin erfindungsgemäß sind Topologieäquivalente Peptide oder chemische Strukturen. Entscheidend ist in jedem Fall die Möglichkeit die oben definierten Anti-Angiotensin Antikörper zu binden. Besonders bevorzugt für die erfindungsgemäße Verbindung ist Angiotensin I.

Die genannten Angiotensine sind an eine Festphase gebunden. Die Bindung kann dabei N-terminal und/oder C-terminal sein, kann aber auch durch eine der anderen, weiter oben beschriebenen Bindungsmöglichkeiten realisiert sein.

Als Festphase zur Bindung der Angiotensine können die aus dem Stand der Technik für vergleichbare Verfahren bekannten Festphasen herangezogen werden, insbesondere
- Gold-Nanopartikel
- Siliziumdioxid
- Glasperlen (beads)
- Kohlenstoffkügelchen (beads)
- Metallkügelchen (beads)
- Magnetische Beads
- Polystyrolderivat
- Polykarbonatderivate
- Poly(vinylchlorid)
- Polyacrylamid
- Polyethylenterephthalat (PET)
- Polyvinylidenfluorid (PVDF)-Derivate
- Polyvinylacetat (PVAc)-Derivate
- Polyvinylpyrrolidon (PVP)-Derivate
- Polyethylenoxid (PEO)-Derivate
- Poly(ethylenimin)-Derivate (PEI)
- NHS-aktivierte Agarose / Sepharose / Hydrogele
- Agarose / Sepharose / Hydrogele gegebenenfalls mit immobilisiertem DADPA oder NHS
- (CarboxyLink-Harz)

Die Festphasen können oberflächlich modifiziert werden, um Spacer oder Angiotensine daran zu binden (z. B. Thiol-modifiziert, Amin-modifiziert). Als Festphase besonders bevorzugt für die vorliegende Erfindung sind Alginate und Hydrogele.

Die Verfahren zum Binden von Angiotensinen an eine Festphase sind im Stand der Technik ausreichend beschrieben, die notwendigen Materialien und Reagenzien sind größtenteils handelsüblich, so dass Erläuterungen an dieser Stelle entbehrlich sind.

Zwischen der Festphase und dem Angiotensin muss ein Linker vorhanden sein zur Bindung des Angiotensins mit der Festphase. Der Begriff Linker umfasst für die Zwecke der vorliegenden Erfindung auch Gruppen, die gelegentlich als "Anchor" bezeichnet werden und zur Bindung von Peptiden und/oder Spacer an die Festphase dienen. Derartige Linker sind aus dem Stand der Technik bekannt. Beispielhaft genannt seien folgende Linker:
- Peptidderivate (z. B. PEG-Peptid, PVA-Peptid)
- Disulfidbindungen enthaltende Verbindungen (z. B. BMPER)
- Derivate mit aminreaktiven Gruppen (z. B. DCC, DBCO)
- Derivate mit Carbonyl-reaktiven Gruppen (z. B. DCC, DBCO)
- Derivate mit thiolreaktiven Gruppen (z. B. DTSSP, DTSSL)
- DADPA (Diaminodipropylamine)
- Hydrazidhaltige Verbindungen
- Derivate von Hydroxysuccinimidestern (z. B. DBCO, BMPER)
- Derivate mit reaktiven Carboxylgruppen (z. B. EDC, NHS)
- Derivate mit Epoxy-reaktiven Gruppen (z. B. EDC, NHS)
- Derivate der Klick-Chemie (z. B. Alkin-Azid)
- Derivate mit photolabilen Gruppen (z. B. DMTP)
- Peptide (z. B. Gly-Gly-Cys)
- Disulfidbindungen
- Amin-reaktive Gruppen (z. B. NHS-Ester)
- Carbonyl-reaktive Gruppen (z. B. NHS-Amide)
- Thiol-reaktive Gruppen (z. B. Maleimide)
- Hydrazid
- Hydroxysuccinimid-Ester (NHS-Ester)
- Carboxyl-reaktive Gruppen (z. B. EDC)
- Epoxid-reaktive Gruppen (z. B. EDC)
- Photolabile Gruppen/Proteine (z. B. DMT, MeNPOC, NPPOC) siehe auch Weiterführende Literatur, unten)
- Klick-Chemie (z. B. Azid-Alkin-Cycloaddition)
   Erläuterung zur Klick-Chemie: Bei der Klick-Chemie werden chemische Reaktionen wie die kupferkatalysierte Azid-Alkin-Cycloaddition (CuAAC) oder die spannungsunterstützte Azid-Alkin-Cycloaddition (SPAAC) eingesetzt, um kovalente Bindungen zwischen dem Protein und der Oberfläche herzustellen.

Besonders bevorzugte Linker sind Lektine sowie Biotin/Avidin bzw. Streptavidin.

Zur Vermeidung der Kontamination der Körperflüssigkeiten bei der extrakorporalen Wäsche durch Angiotensine ist die Bindung zwischen Festphase und Angiotensin bzw. Festphase und Spacer sowie zwischen Spacer und Angiotensin bevorzugt als kovalente Bindung ausgestaltet.

Die Verfahren zum Koppeln der Angiotensine an eine Festphase mittels eines Linkers sind im Stand der Technik ausreichend beschrieben und bedürfen an dieser Stelle keiner Erläuterung.

Zwischen der Festphase und dem Angiotensin kann zusätzlich ein Spacer vorhanden sein. Ein Spacer ist im Allgemeinen ein Molekül oder eine Gruppe von Molekülen, die das Protein von der Festphase trennt und einen Abstand zwischen dem Protein und der Festphasenoberfläche bildet. Geeignet hierfür sind insbesondere starre oder flexible Peptide oder Proteine. Aus dem Stand der Technik sind zahlreiche weitere Spacer bekannt, die ohne Weiteres verwendet werden können.

Als Spacer beispielhaft genannt seien:
- Polyethylenglykol (PEG)-Derivate (z. B. PEG-Thiol, PEG-Amin)
- Polyvinylalkohol (PVA)-Derivate (z. B. PVA-Thiol, PVA-Amin)
- Polysaccharidderivate (z. B. Dextransulfat, Chitosan)
- Polyamidoamin (PAMAM)-Dendrimere
- Polyamidoxim-Dendrimere (PAOx)
- Polyethylenimin (PEI)-Derivate
- Polyethylenoxid (PEO)-Derivate
- Polyvinylpyrrolidon (PVP)-Derivate
- Polyvinylacetat (PVAc)-Derivate
- Polyvinylchlorid (PVC)-Derivate
- Polyvinylidenfluorid (PVDF)-Derivate
- Polyacrylamid (PAAm) Derivate
- Derivate von Polymethacrylat (PMMA)
- Polyvinylpyrrolidon (PVP)
- Polyethylenoxid (PEO)
- Poly(ethylenimin) (PEI)
- Polyvinylalkohol (PVA)
- Dextran

Das gesamte Verfahren der Herstellung der erfindungsgemäßen Vorrichtung kann im Prinzip so durchgeführt werden, wie es für andere Peptide als Antikörperfänger in ähnlichen Anwendungen beschrieben ist, z.B. in DE 1953864 A1.

Es ist bekannt, dass Linker durch im Blutplasma enthaltene Proteasen abgebaut werden können. Zur Vermeidung derartiger Abbauvorgänge können die genannten Linker durch N-terminale und/oder C-terminale Cappings vor dem Abbau durch Proteasen geschützt werden.

Bekannte Cappings sind nachfolgend aufgezählt:

### A) N-Terminale Cappings:

1 Acetylierung - Anfügung einer Acetylgruppe an die Aminogruppe des N-Terminus eines Proteins, was die Stabilität, Lokalisierung und Funktion des Proteins beeinflussen kann.
2 Formylierung - Anfügung einer Formylgruppe an den N-Terminus eines Proteins, die bei bakteriellen Proteinen auftritt und die Immunerkennung des Proteins beeinträchtigen kann.
3 Myristoylierung - Anfügung eines Myristinsäuremoleküls an den N-Terminus eines Proteins, was die Membranassoziation und die Signalübertragung beeinflussen kann.
4 Palmitoylierung - Anfügung eines Palmitinsäuremoleküls an den N-Terminus eines Proteins, was die Membranassoziation und -stabilität beeinflussen kann.
5 Glykosylierung - Anfügung eines Zuckermoleküls an den N-Terminus eines Proteins, was die Faltung, Stabilität und Funktion beeinflussen kann.
6 Phosphorylierung - Anfügung einer Phosphatgruppe an den N-Terminus eines Proteins, was sich auf Signalübertragung, Aktivität und Lokalisierung auswirken kann.
7 Methylierung - Anfügung einer Methylgruppe an den N-Terminus eines Proteins, was sich auf Protein-Protein-Interaktionen und die Transkriptionsregulation auswirken kann.
8 Sumoylierung - Hinzufügung eines kleinen Ubiquitin-ähnlichen Modifikators (SUMO) an den N-Terminus eines Proteins, was Protein-Protein-Wechselwirkungen und die Lokalisierung beeinflussen kann.
9 Ubiquitinierung - Anhängen von Ubiquitin an den N-Terminus eines Proteins, was den Proteinabbau, die Lokalisierung und die Signalübertragung beeinflussen kann.
10 Acylierung - Anfügung einer Acylgruppe (wie Acetyl, Palmitoyl oder Myristoyl) an den N-Terminus eines Proteins, was die Membranassoziation und -stabilität beeinflussen kann.
11 N-terminale Trunkierung - Entfernung der N-terminalen Aminosäuren aus einem Protein, was die Faltung, Stabilität und Aktivität des Proteins beeinträchtigen kann.
12 Proteolytische Spaltung - enzymatische Entfernung einer Peptidbindung am N-Terminus eines Proteins, wodurch das Protein aktiviert oder inaktiviert werden kann.
13 Oxidation - Anlagerung eines Sauerstoffmoleküls an den N-Terminus eines Proteins, was die Stabilität und Aktivität beeinflussen kann.
14 Sulfatierung - Anfügung einer Sulfatgruppe an den N-Terminus eines Proteins, was die Protein-Protein-Interaktionen und die Funktion beeinträchtigen kann.
15 Nitrosylierung - Anfügung eines Stickstoffmonoxidmoleküls an den N-Terminus eines Proteins, was sich auf die Proteinaktivität und die Signalübertragung auswirken kann.
16 Glutathionylierung - Anlagerung eines Glutathionmoleküls an den N-Terminus eines Proteins, was Protein-Protein-Wechselwirkungen und -funktion beeinträchtigen kann.
17 Prolin-Isomerisierung - Isomerisierung des N-terminalen Prolinrestes eines Proteins, die die Proteinfaltung und -stabilität beeinträchtigen kann.
18 Formylierungsabhängige Initiierung - Initiierung der Translation mit einem formylierten Methioninrest am N-Terminus eines Proteins, die bei bakteriellen Proteinen auftritt.
19 Pyroglutamatbildung - Bildung eines Pyroglutamatrestes am N-Terminus eines Proteins, der die Proteinstabilität und -funktion beeinflussen kann.

### B) C-Terminale Cappings:

1 Amidierung - Umwandlung der C-terminalen Carboxylgruppe eines Proteins in eine Amidgruppe, was die Stabilität, Aktivität und Lokalisierung des Proteins beeinflussen kann.
2 Glykosylierung - Anfügung eines Zuckermoleküls an den C-Terminus eines Proteins, was sich auf Faltung, Stabilität und Funktion auswirken kann.
3 Phosphorylierung - Anfügung einer Phosphatgruppe an den C-Terminus eines Proteins, was sich auf die Signalübertragung, die Aktivität und die Lokalisierung auswirken kann.
4 Methylierung - Anfügung einer Methylgruppe an den C-Terminus eines Proteins, was sich auf Protein-Protein-Interaktionen und die Transkriptionsregulation auswirken kann.
5 Sumoylierung - Anfügung eines kleinen ubiquitinähnlichen Modifikators (SUMO) an den C-Terminus eines Proteins, was Protein-Protein-Interaktionen und die Lokalisierung beeinflussen kann.
6 Ubiquitinierung - Anhängen von Ubiquitin an den C-Terminus eines Proteins, was den Proteinabbau, die Lokalisierung und die Signalübertragung beeinflussen kann.
7 C-terminale Trunkierung - Entfernung der C-terminalen Aminosäuren aus einem Protein, was die Faltung, Stabilität und Aktivität des Proteins beeinflussen kann.
8 Proteolytische Spaltung - enzymatische Entfernung einer Peptidbindung am C-Terminus eines Proteins, wodurch das Protein aktiviert oder inaktiviert werden kann.
9 Sulfatierung - Hinzufügung einer Sulfatgruppe am C-Terminus eines Proteins, was die Protein-Protein-Wechselwirkungen und die Funktion beeinflussen kann.
10 Nitrosylierung - Anfügung eines Stickstoffmonoxidmoleküls an den C-Terminus eines Proteins, was die Proteinaktivität und die Signalübertragung beeinflussen kann.
11 Glutathionylierung - Anlagerung eines Glutathionmoleküls an den C-Terminus eines Proteins, was Protein-Protein-Wechselwirkungen und -funktion beeinträchtigen kann.
12 Ribosomales Skipping - Überspringen der C-terminalen Aminosäuren während der Translation, was die Aktivität und Stabilität von Proteinen beeinträchtigen kann.
13 Prolin-Isomerisierung - Isomerisierung des C-terminalen Prolin-Restes eines Proteins, die die Proteinfaltung und -stabilität beeinträchtigen kann.
14 Peptidylarginin-Deiminase (PAD)-Modifikation - Umwandlung von Argininresten in Citrullinreste am C-Terminus eines Proteins durch PAD-Enzyme, was die Proteinfunktion und Immunerkennung beeinträchtigen kann.
15 Glutaminsäure-Decarboxylierung - Decarboxylierung des C-terminalen Glutaminsäurerests eines Proteins, was die Aktivität und Funktion des Proteins beeinträchtigen kann.
16 Asparagin-Desamidierung - Deamidierung des C-terminalen Asparagin-Rests eines Proteins, was die Funktion und Stabilität des Proteins beeinträchtigen kann.
17 Lysin-Carboxylierung - Anfügung einer Carbonsäuregruppe an den C-terminalen Lysinrest eines Proteins, was Protein-Protein-Wechselwirkungen und -funktion beeinträchtigen kann.
18 Tyrosinsulfatierung - Anlagerung einer Sulfatgruppe an den C-terminalen Tyrosinrest eines Proteins, was Protein-Protein-Wechselwirkungen und die Funktion beeinträchtigen kann.
19 C-terminale Thioveresterung - Anfügung einer Thiolgruppe an die C-terminale Carboxylgruppe eines Proteins, was die Protein-Protein-Wechselwirkungen und die Funktion beeinflussen kann.

Die Verfahren zum Cappen von Linkern sind im Stand der Technik ausreichend beschrieben und bedürfen an dieser Stelle keiner Erläuterung.

Zur erfindungsgemäßen Anwendung sind die Festphasen-gekoppelten Angiotensine in einer Kartusche angeordnet. Derartige Kartuschen sind bereits aus dem Stand der Technik für andere Anwendungen, insbesondere Blutwäschen (Apheresen), bekannt und sind handelsüblich. Die Kartuschen sind üblicherweise aus Kunststoff, Glas oder einem neutralen Metall gefertigt, sind zylinderförmig ausgestaltet und haben eine Größe 20 bis 150 ml. Die Kartuschen haben an den Stirnseiten zwei Anschlüsse und können in die üblichen Vorrichtungen zur Blutwäsche integriert werden. Die Kartuschen können insbesondere am Eingang Diffusoren aufweisen, die die Strömung in der Kartusche gleichmäßig verteilen und turbulent durchmischen. Selbstverständlich können bei Bedarf mehrere Kartuschen nacheinander verwendet werden. Ebenso können mehrere Kartuschen parallel betrieben werden, um einen höheren Durchsatz des Systems zu ermöglichen.

Eine erste Ausführungsform der Erfindung ist eine Blutwäsche: Zur Durchführung der extrakorporalen Blutwäsche wird zunächst aus dem betroffenen Patienten Blut entnommen. Aus dem entnommenen Vollblut wird das Blutplasma extrahiert und durch die Kartusche geleitet, in der sich die Festphasen-gebundenen Angiotensine befinden. Beim Durchlaufen durch die Kartusche binden sich die Anti-Angiotensin Antikörper an die Angiotensine. Das aus der Kartusche austretende Blutplasma ist daher im Wesentlichen frei von diesen Anti-Angiotensin Antikörpern. Das Plasma wird mit dem im ersten Schritt abgetrennten anderen Blutbestandteilen (z. B. Erythrozyten) zusammengeführt und wieder in den Patienten zurückgeführt. Dieses Verfahren kann als kontinuierlicher Prozess ausgestaltet werden. Der Patient wird dabei typischerweise zwei bis fünf Stunden der beschriebenen extrakorporalen Blutwäsche unterzogen.

Nach der beschriebenen extrakorporalen Blutwäsche ist der Patient zunächst frei von den eingangs beschriebenen Anti-Angiotensin Antikörpern. Die durch die Antikörper ausgelösten Nebenwirkungen lassen typischerweise nach, insbesondere das in der Einleitung beschriebene Renin-Angiotensin-Aldosteron-Systems (RAAS) pendelt sich wieder in den Normalzustand ein.

Erforderlichenfalls kann das Verfahren mehrfach wiederholt werden.

Eine zweite Ausführungsform der Erfindung ist eine Liquorwäsche. Zur Durchführung der extrakorporalen Liquorwäsche wird zunächst aus dem betroffenen Patienten Liquor *(Liquor cerebrospinalis*) z.B. aus dem Subarachnoidalraum oder dem Spinalkanal mittels einer Lumbalpunktion entnommen. Der Liquor wird durch die Kartusche geleitet, in der sich die Festphasen-gebundenen Angiotensine befinden. Beim Durchlaufen durch die Kartusche binden sich die spezifischen Antikörper an die Angiotensine. Der aus der Kartusche austretende Liquor ist daher im Wesentlichen frei von diesen Anti-Angiotensin Antikörper und wird wieder in den Patienten zurückgeführt. Dieses Verfahren kann als kontinuierlicher Prozess ausgestaltet werden. Der Patient wird dabei typischerweise zwei bis fünf Stunden der beschriebenen extrakorporalen Liquorwäsche unterzogen. Erforderlichenfalls kann das Verfahren mehrfach wiederholt werden.

Die beschriebenen Prozeduren ähneln anderen Immunadsorptions-Verfahren, wie sie beispielsweise zur Entfernung von Autoimmun-Antikörpern aus dem Körper durchgeführt werden.

Die nach Durchführung des Verfahrens mit den Angiotensin-spezifischen Antikörpern beladenen Kartuschen können optional einer Reinigung unterzogen werden. Hierzu werden die entweder die gebundenen Antikörper durch fachübliche Verfahren von den Angiotensinen abgetrennt oder die Bindung des Linkers wird gelöst unter Abtrennung der Angiotensine. Hierzu können beispielsweise chaotrope Salze und/oder Detergentien eingesetzt werden. Bei Nutzung eines Biotin/Avidin bzw. Streptavidin Linkers besteht auch die Möglichkeit, mit eine Biotin-Lösung zu spülen, wobei die Lösung eine 10-100fach höhere Konzentration als das gebundene Biotin aufweist. Bei Bindungen zwischen Kohlehydraten und Lektin können Kohlehydratlösungen die Bindung durch Konkurrenzreaktionen spalten. Alternativ oder ergänzend können pH-Änderungen oder chaotrope Salze zur Ablösung führen. Bei Verwendung von Licht-dissoziierbaren Linkern, kann die Freisetzung durch Lichteinstrahlung erfolgen. Die einzelnen Prozeduren sind fachüblich und sollen daher an dieser Stelle nicht weiter erläutert werden.

Nach einer abschließenden Wäsche kann die Kartusche wieder eingesetzt werden.

### Beschreibung der Figuren

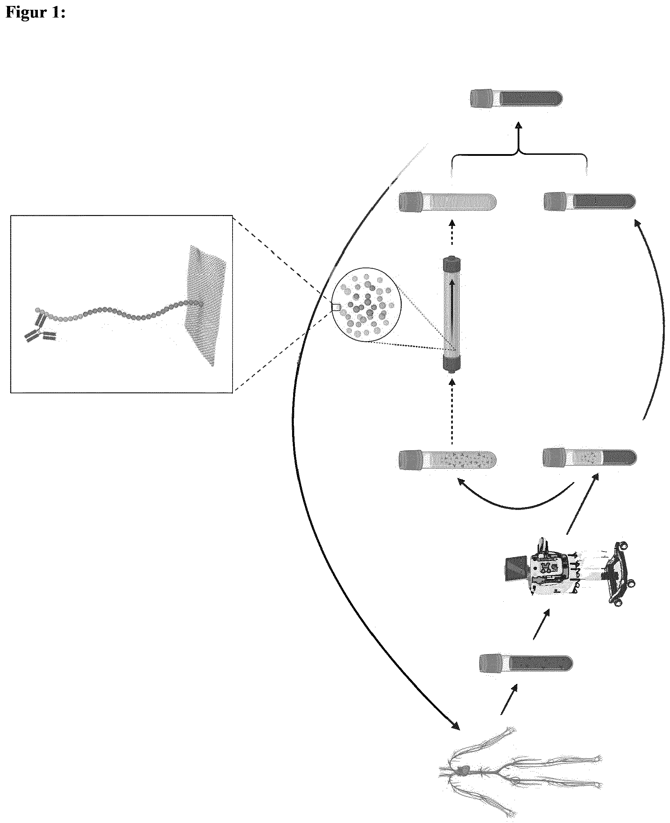

**Figur 1** zeigt schematisch die erste Anwendung der erfindungsgemäßen Vorrichtung: Zunächst wird dem betroffenen Patienten Blut entnommen. Aus dem entnommenen Vollblut wird das Blutplasma extrahiert und durch die Kartusche geleitet, in der sich die Festphasen-gebundenen Angiotensine befinden. Das Plasma wird mit dem im ersten Schritt abgetrennten anderen Blutbestandteilen (z. B. Erythrozyten) zusammengeführt und wieder dem Patienten zugeführt. Dieses Verfahren kann als kontinuierlicher Prozess ausgestaltet werden.

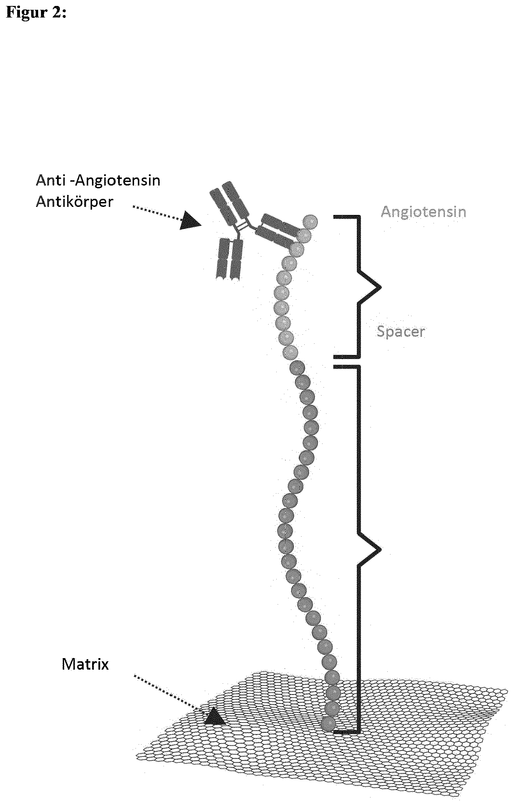

**Figur 2** zeigt schematisch das festphasengebundene Angiotensin, welches mittels eines Spacers an die Festphase (Alginat-Matrix) gebunden ist. Ebenfalls schematisch dargestellt ist der gebundene Antikörper.

### Weiterführende Literatur

**Generelle Publikationen zur Proteinimmobilisierung**
   https://www.sciencedirect.com/topics/biochemistry-genetics-and-molecularbiology/protein-immobilization.
**Publikationen für Linker**
   https://broadpharm.com/product-categories/adc-linkers
   https://www.pepscan.com/custom-peptide-synthesis/peptide-modifications/linkers-spacers/
   https://www.researchgate.net/profile/Ziliang-Huang-5/publication/282125989 Construction of a linker library with widely controllable flexi bility for fusion protein design/links/56203f1008ae70315b552a41/Construction-of-a-linker-library-with-widely-controllable-flexibility-for-fusion-protein-design.pdf?origin=figuresDialog download& rtd=e30%3D
   https://www.sciencedirect.com/science/article/abs/pii/S0076687920303700
   https://www.kbdna.com/publishinglab/lnkr
   https:!/pubs.acs.org/doi/pdf/10.1021/bm061197b
   https://www.sciencedirect.com/science/article/pii/S0141022915300752?via%3Dihub
**Datenbanken für Linker**
   https://www.ibi.vu.nl/programs/linkerdbwww/
   http://parts.igem.org/Protein domains/Linker
**Lectin Bindung:**
   https:! /www.freepatentsonline.com/DE102016011057.htm I
   https://www.researchgate.net/publication/51657008 Lectin Microarrays A Powerful Tool for Glycan-Based Biomarker Discovery/figures?lo=1
**Photolabile Linker**
   Licht-dissoziierbare syntaktische Organellen/ Licht-dissoziierbare optogenetische Proteine, siehe z.B.: Schematic of optoCluster and PixELL formation; Zhao et al. (2019) Verfahren
**Immobilisierung mittels NHS-aktivierter Agarose**
   https://de.vwr.com/store/product/18502975/nhs-activated-agarose-piercetm
**Immobilisierung mittels Diamino-Dipropylamin-(DADPA)-aktivierter Agarose**
   https://www.thermofisher.com/order/catalog/product/20266
**Autoimmunadsorptionsverfahren**
   https://www.freseniusmedicalcare.com/fileadmin/data/de/pdf/Healthcare_Professionals/IA /IA_Therapiebroschuere_Immunadsorption_03_14_D.pdf

## Patentansprüche

1. Vorrichtung zur extrakorporalen Wäsche von Körperflüssigkeiten durch Abtrennung von Angiotensin-spezifischen Antikörpern aus der Körperflüssigkeit, enthaltend mindestens eine Kartusche mit festphasengebundenen Angiotensinen.

2. Vorrichtung zur extrakorporalen Wäsche von Körperflüssigkeiten gemäß Anspruch 1, wobei die Angiotensine Angiotensin II, Angiotensinogen, Angiotensin I, Angiotensin 1-9 und/oder Angiotensin 1-7 umfassen.

3. Vorrichtung zur extrakorporalen Wäsche von Körperflüssigkeiten gemäß Anspruch 1, wobei die Angiotensine durch Linker an eine Festphase gebunden sind.

4. Vorrichtung zur extrakorporalen Wäsche von Körperflüssigkeiten gemäß Anspruch 1, wobei ein Spacer zwischen den Angiotensine und der Festphase angeordnet ist.

5. Vorrichtung zur extrakorporalen Wäsche von Körperflüssigkeiten gemäß Anspruch 1, wobei die Linker durch N-terminale und/oder C-terminale Cappings vor dem Abbau durch Proteasen geschützt sind.

6. Vorrichtung zur extrakorporalen Wäsche von Körperflüssigkeiten gemäß Anspruch 1, wobei die Angiotensine N-terminal und/oder C-terminal gebunden sind.

7. Vorrichtung zur extrakorporalen Wäsche von Körperflüssigkeiten gemäß Anspruch 1, enthaltend eine Alginat Festphase, einen Peptidspacer, wobei der Peptidspacer durch NHS oder DADPA gebunden ist sowie an den Peptidspacer gekoppeltes Angiotensin I.

8. Verfahren zur extrakorporalen Wäsche von Körperflüssigkeiten durch Abtrennung von Angiotensin-spezifischen Antikörpern aus dem Blutplasma mit folgenden Schritten:
a) Entnahme von Vollblut aus einem Patienten
b) Abtrennung des Blutplasmas aus dem Vollblut
c) Durchleitung des Blutplasmas durch mindestens eine Kartusche enthaltend festphasengebundene Angiotensine
d) Wiedervereinigung des gereinigten Blutplasmas mit den in Schritt b abgetrennten Blutbestandteilen
e) Rückführung des gereinigten Vollblutes zum Patienten.

9. Verfahren zur extrakorporalen Wäsche von Körperflüssigkeiten durch Abtrennung von Angiotensin-spezifischen Antikörpern aus dem Blutplasma gemäß Anspruch 8, wobei das Verfahren als kontinuierliches Verfahren ausgestaltet ist.

10. Verfahren zur extrakorporalen Wäsche von Körperflüssigkeiten durch Abtrennung von Angiotensin-spezifischen Antikörpern aus dem Liquor mit folgenden Schritten:
a) Entnahme von Liquor aus dem Subarachnoidalraum oder dem Spinalkanal eines Patienten
b) Durchleitung des Liquors durch mindestens eine Kartusche enthaltend festphasengebundene Angiotensine
e) Rückführung des gereinigten Liquors zum Patienten.

11. Verfahren zur extrakorporalen Wäsche von Körperflüssigkeiten durch Abtrennung von Angiotensin-spezifischen Antikörpern aus dem Liquor gemäß Anspruch 10, wobei das Verfahren als kontinuierliches Verfahren ausgestaltet ist.
